# EUROPEAN PATENT APPLICATION

(11) **EP 1 985 314 A1**
(43) Date of publication of application: **29.10.2008**
(21) Application number: 08007711.8
(22) Date of filing: 21.04.2008
(51) Int. Cl.: A61L 2/18, A61L 2/26

(54) **Endoscope washer disinfector and brush cassette with a washing brush used for wash and disinfection**

(30) Priority: 19.04.2007 JP 2007110773
(71) Applicant: Olympus Medical Systems Corp., Tokyo 151-0072 (JP)
(72) Inventor: Onishi, Hideto, Tokyo 151-0072 (JP)
(74) Representative: von Hellfeld, Axel

(57) **Abstract**

A brush cassette is detachably loaded to an endoscope washer dlsinfector for washing and disinfecting an endoscopic duct of an endoscope. The brush cassette comprises a washing brush, a chamber accommodating the washing brush, and a brush delivery duct that allows the washing brush to pass therethrough toward the duct of the endoscope. Further the brush cassette comprises a fluid supply duct that allows a chemical to pass therethrough toward the chamber; a drive member used for moving back and forth the washing brush between the chamber and the duct via the brush delivery duct; a means that prevents air from accumulating in the chamber; and a remaining-fluid discharge duct that allows the chemical remaining in the chamber to be discharged outside therethrough. The preventing means is a duct discharging the air or a deflection member deflecting the chemical toward a region in which the air is to accumulate.

## Description

### CROSS REFERENCE TO RELATED APPLICATION

The patent application related to and incorporates by reference Japanese Patent Application No. 2007-110773 filed on April 19, 2007.

### Background of the Invention

### (The field of the Invention)

The present invention relates to an endoscope washer disinfector used for washing and disinfecting endoscopes and a brush cassette in which a washing brush for wash and disinfection is mounted insertably into endoscopic channels.

### (Related art)

In the medical field, endoscopes are used for examining and treating the insides of cavities of an object being examined, so medical endoscopes have now become one of indispensable medical modalities. Once an endoscope is used, not only the outer surface of the insertion tube of the endoscope but also the inside thereof, that is, its endoscopic ducts (including an air-supply water-supply duct, a suction duct, a forward water-supply duct, and an therapeutic-instrument insertion duct; hereinafter referred to as channels), become contaminated because of waste materials such as bodily fluid.

Thus, it is absolutely necessary for used endoscopes to wash and disinfect the outer surface and endoscopic ducts thereof. To meet such a demand, an endoscope washer disinfector (that Is, an apparatus for washing and disinfecting endoscopes) which Is capable of automatically washing and disinfecting an endoscope, including its endoscopic channels, has already been put to practical use.

Of the endoscopic channels, in particular, the suction channel and the therapeutic-instrument insertion channel are used such that pieces of tissue or others collected in body cavities pass therethrough. Hence, there channels are liable to be contaminated by waste materials which are difficult to be cleared away.

To raise the washed performance of the channels of the endoscope, it is usual that a user preliminarily washes the endoscopic channels by hand, prior to the washing and disinfection processes carried out by the endoscope washer disinfector. Specifically, the user uses a washing brush to rub up endoscopic channels with the brush secured at the tip of a long wire inserted into the endoscopic channels. Thus larger-size waste materials on the endoscopic channels can be cleared away.

However, this preliminary wash Is considerably troublesome work for the user, making the washing work time longer

In consideration of this drawback, the apparatus for automatically washing and disinfecting endoscopes is used. In this apparatus, wash fluid and disinfection fluid are supplied and a washing brush is automatically inserted into an endoscopic duct (channel), and the washing brush is driven to move back and forth through the duct. Thus the endoscopic duct can be washed with sliding motions of the brush therethrough.

This kind of apparatus is provided with a brush cassette, which has a brush containing chamber with a washing brush wound and contained therein and rotary rollers for driving the washing brush. Since the rotary rollers engage with an electric motor positioned in the apparatus body, the rotary rollers can be rotated by the motor. When the motor rotates the rollers, it is possible to deliver the washing brush from the chamber to the endoscopic channel or pull back the washing brush from the endoscopic channel to the chamber.

Brush cassettes include disposal type of brush cassettes and reusable type of brush cassettes. When the reuse type of brush cassette is used, the wash and disinfection operations are carried out, while chemicals such as wash fluid and disinfection fluid are supplied to the washing brush contained in the brush containing chamber during the wash and disinfection. Thus, without removing the brush cassette from the endoscope washer disinfector, the brush cassette can be used predetermined plural times.

For using this reuse type of brush cassette, it is also necessary to supply the chemicals from the chamber to the endoscopic duct via a delivery duct connected to the chamber. Concurrently the washing brush should be washed and disinfected in the chamber: In addition to the delivery duct for the washing brush, a fluid supply duct should also be connected to the brush containing chamber for supplying the chemicals to this chamber. This fluid supply duct is exemplified by Japanese Patent Application Laid-open Publication No. 2002-209847, where a duct to supply chemicals to a wire containing chamber is disclosed.

By the way, the foregoing brush containing chamber contains a wound washing brush. The chamber needs to have an inner volume suitable for containing the washing brush. Compared to the radius of the delivery duct for the washing brush, the volume of inner space of the brush containing chamber is larger, with the result that air which has been mixed with the chemicals accumulates in an upper region of the chamber during the supply of the chemicals to the chamber via the fluid supply duct. This is called "air accumulation" or "accumulated air."

When this air accumulation occurs, a portion of the washing brush, which is located at the air accumulation, may not be completely immersed (not-touch to the chemicals) or may be partially immersed (insufficiently touch the chemicals). These events may cause poor disinfection or wash performance in the wash performance or the disinfection performance for the washing brush. Thus when such an air accumulation is found in the chamber, the brush cassette should be removed to discharge the air or to wash and disinfect the washing brush using another means. This is very troublesome for operators, lowering the efficiency of the wash and disinfection work. In addition, to monitor the air accumulation at any time is a heavier burden on the operators.

In addition to the drawback from the air accumulation, the conventional brush cassette faces another difficulty resulting from a chemical which remains in the chamber A chemical supplied into the brush containing chamber via the fluid supply duct is further fed to an endoscopic duct via the delivery duct. That is, the chemical is discharged outside of the brush containing chamber. Since the delivery duct is formed at a certain height within the chamber, a certain amount of chemical, which has not been discharged from the delivery duct, will remain or is likely to remain on the bottom of the chamber. Accordingly, when the disposal type of brush cassette is removed, the remaining chemical may leak out abruptly from the delivery duct. It is therefore for operators to pay sufficient attention to the removal work of the brush cassette in terms of leaking out the remaining chemical. This is also a factor to reduce the efficiency of the wash and disinfection work.

### Summary of the Invention

The present invention has been made in consideration of the foregoing various problems, and an object of the present invention is to provide a brush cassette and an endoscope washer disinfector with the brush cassette, which are able to supply chemicals to all the portions of the washing brush contained in the brush containing chamber for a reliable wash and disinfection of the washing brush and to prevent the chemicals from remaining in the chamber after the chemicals are discharged from the chamber.

In order to realize the above object, there is provided a brush cassette detachably loaded to an apparatus that provides a bath for washing and disinfecting a duct of an endoscope accommodated in the bath. The brush cassette comprises: a washing brush; a chamber accommodating the washing brush; a brush delivery duct that communicates with the chamber and allows the washing brush to pass therethrough toward the duct of the endoscope; a fluid supply duct that communicates with the chamber and allows a chemical for washing and disinfecting the duct to pass therethrough toward the chamber; a brush drive member used for moving back and forth the washing brush between the chamber and the duct of the endoscope via the brush delivery duct; a preventing means that prevents air from accumulating in the chamber; and a remaining-fluid discharge duct that communicates with the chamber and allows the chemical remaining in the chamber to be discharged outside therethrough.

The present invention also provides an apparatus equipped with the above brush cassette.

In the brush cassette and the apparatus according to the present invention, It is avoided that the air accumulates in the brush containing chamber. It is therefore possible to reliably supply chemicals to every portion of the washing brush contained in chamber. The washing brush can be washed and disinfected in the chamber without failure. Additionally, the remaining chemicals can be discharged through the remaining-fluid discharged duct when the chemical supply ends. This facilitates operator's removal work of the brush cassette from the apparatus body.

### Brief Description of the Drawings

In the accompanying drawings:
Fig. 1 is a perspective view showing the appearance of an endoscope washer disinfector according to an example of the present invention, with a top cover thereof opened;
Fig. 2 is a perspective view showing the endoscope washer disinfector with the top cover closed;
Fig. 3 is a plan view schematically showing the main body of the endoscope washer disinfector;
Fig. 4 is a perspective view showing the appearance of the main unit of a brush cassette detachably installed in the endoscope washer disinfector;
Fig. 5A is a sectional view of the brush cassette, which is taken along a V-V line in Fig. 4;
Fig. 5B is a sectional view of a brush cassette according to a modification of the brush cassette shown in Fig. 5A;
Fig. 6 is a perspective view showing a brush cassette according to another modification;
Fig. 7 is a perspective view showing a brush cassette according to another modification;
Fig. 8 is a perspective view showing a brush cassette according to another modification;
Fig. 9 is a perspective view showing a brush cassette according to another modification;
Fig. 10 is a perspective view showing, together with an endoscope, a brush cassette according to another modification;
Fig. 11 is a perspective view showing a brush cassette according to another modification;
Fig. 12 is a perspective view showing a brush cassette according to another modification; and
Fig. 13 is a perspective view showing an endoscope washer disinfector according to a further embodiment of the present invention.

### Detailed Description of the Preferred Embodiments

Hereinafter, embodiments and their modifications according to the present invention will now be described with referenced to the accompanying drawings.

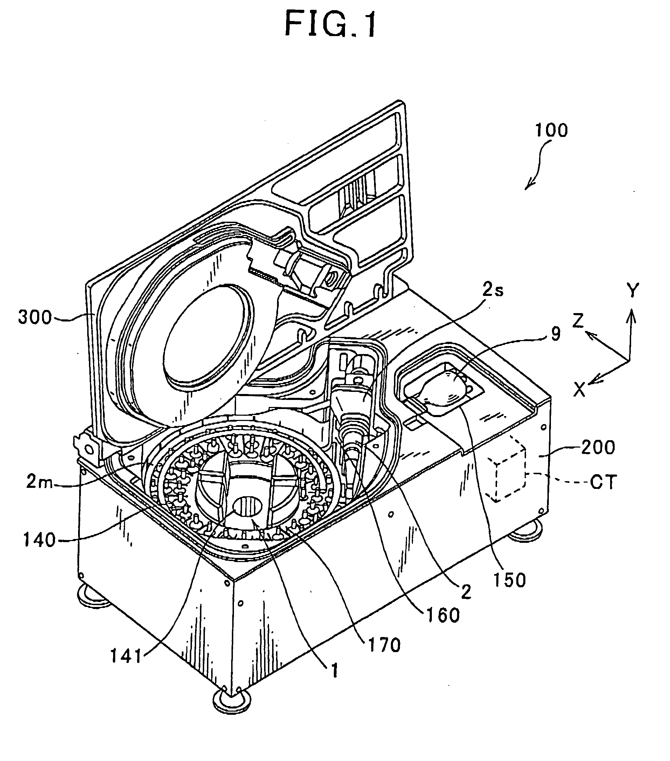

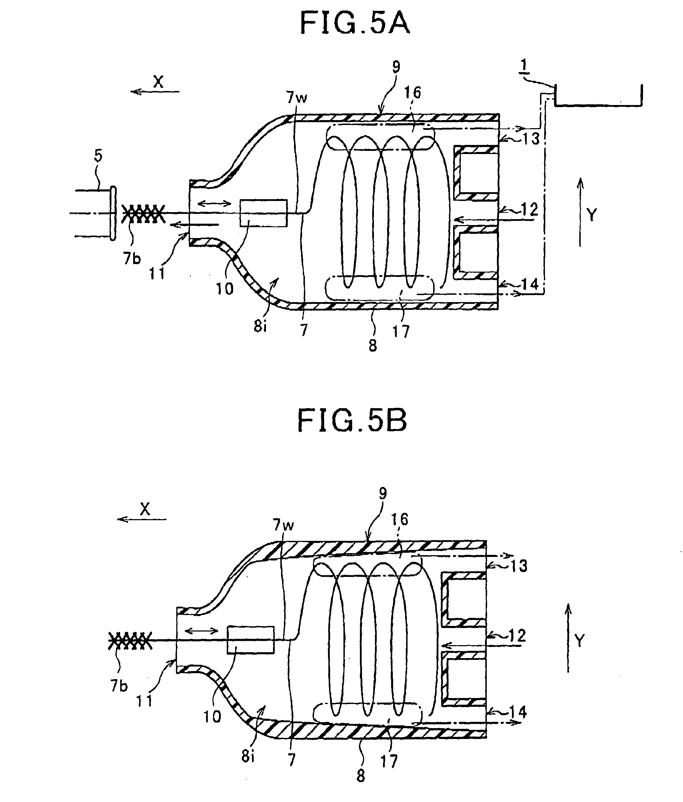

Referring to Figs. 1-5A, 5B, an endoscope washer disinfector (i.e., an apparatus for washing and disinfecting endoscopes) and a brush cassette according to an embodiment of the present invention will now be described.

Fig. 1 shows an endoscope washer disinfector 100 according to the present embodiment. As shown, the endoscope washer disinfector 100 Is provided with an apparatus body 200 with essential components for processing (including washing (cleaning), disinfecting, rinsing, and drying processes) endoscopes and a top cover 300 serving as a lid member which can be opened and closed on the top of the apparatus body 200.

On upper parts of the apparatus body 200, there are formed a washing/disinfecting bath 1 in which an endoscope 2 to be washed and disinfected is mounted and a brush-cassette loading section 150 in which a later-described brush cassette 9 is arranged.

The washing/disinfecting bath 1 has a manipulating-device mounting section 160 formed to comply with the shape of a manipulating device 2s of the endoscope 2 and an insertion-tube mounting section 170 in which an insertion tube 2m of the endoscope 2 is mounted in a wound manner.

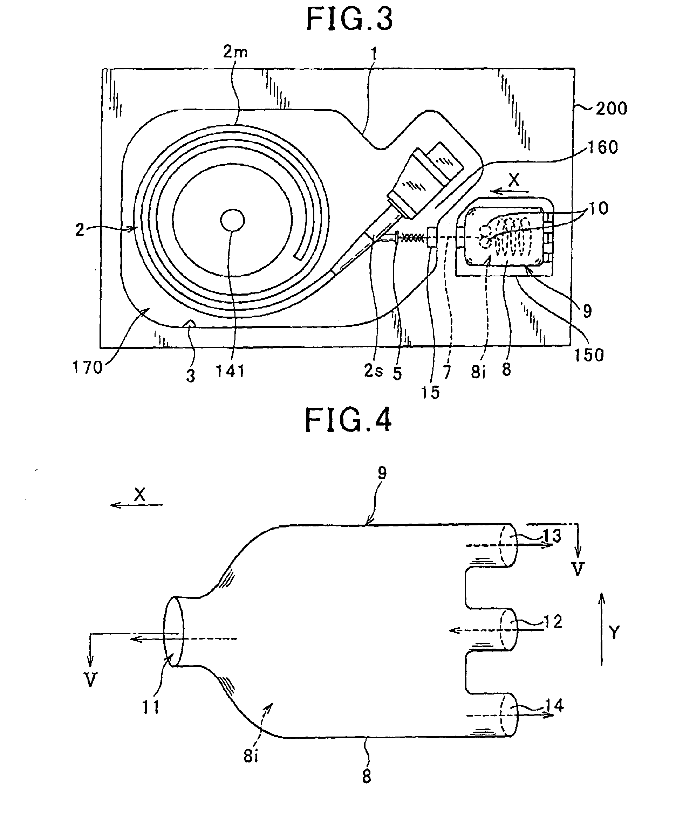

In the insertion-tube mounting section 170, a plurality of holding members 140 are arranged along ring-like tracks when being viewed from above, the holding members 140 holding the round portions of the insertion tube 2m apart from each other. At the center of the ring-like holding members 140 in the insertion-tube mounting section 170, a washing-case mounting section 141 is arranged, to which a not-shown washing case is mounted. The washing case contains buttons, forceps plugs, and others of the endoscope 2 for wash and disinfection together with the endoscope 2. Moreover, as understood from Fig. 3, chemicals such as wash fluid and disinfection fluid are supplied into the insertion-tube mounting section 170 in the washing/disinfecting bath 1, so that the outer surface of the endoscope 2 mounted in the bath 1 is washed and disinfected.

The brush-cassette loading section 150 is formed to be located close to the manipulating-device mounting section 160 of the bath 1. In this loading section 150, the brush cassette 9 is detachable, in an approximately horizontal attitude, on the bottom of the brush-cassette loading section 150. The brush cassette 9 is for washing the endoscopic ducts (channels) of the endoscope 2.

The brush cassette 9 is used to supply, to the endoscopic ducts of the endoscope 2, chemicals such as wash fluid and disinfection fluid and to accommodate a washing brush 7 (refer to Fig. 3). In the present embodiment, this brush cassette 9 is formed as a disposable type of brush cassette, which will be detailed later. The brush cassette 9 may be a re-use type, not limited to the disposal type.

A fluid-supply nozzle 15 is disposed at the brush-cassette loading section 150. The nozzle 15 is located to face an opening 5 (later described; refer to Fig. 3) of a therapeutic-instrument insertion duct (i.e., endoscopic duct) formed through the manipulating device 2s accommodated in the manipulating-device mounting section 160, when the endoscope 2 is mounted in the washing/disinfecting bath 1. Preferably, the fluid-supply nozzle 15 is coaxial to the opening 5. When being automatically loaded to the opening 5, this nozzle 15 is used for, via the opening 5, inserting the washing brush 7 into the therapeutic-instrument insertion duct and supplying the chemicals to the therapeutic-instrument insertion duct. The washing brush 7 comes from the brush cassette 9 and the chemicals are supplied by way of the brush cassette 9.

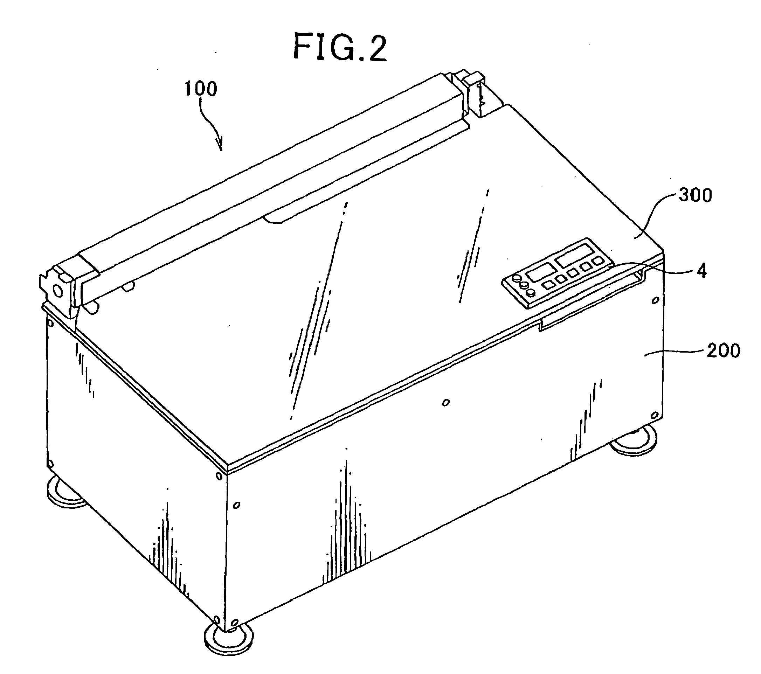

The top cover 300 Is made of an optically transparent or semi-transparent material. Hence, even when the top cover 300 is closed as shown in Fig. 2, it is possible for operators to visually observe the inside of the washing/disinfecting bath 1 and the brush cassette 9.

As shown in Fig. 2, for example, on the upper face of the top cover 300, there is provided an operation panel 4 which is for commanding a start operation, a stop operation, setting washing/disinfecting steps, and others, and displaying progress of various washing/disinfecting steps, elapse time, and others. This operation panel 4 is able to communicate with a controller CT arranged in the apparatus body 200 so that information necessary for the operations of this washer disinfector can be exchanged therebetween. The operation panel 4 may be arranged at a part of the apparatus body 200.

Within the apparatus body 200, there are provided various components including duct networks equipped with electromagnetic valves and check valves, pumps, and compressors, which all are for circulating chemicals and other necessary fluid through washing/disinfecting bath 1 and the bush cassette 9. The apparatus body 200 includes the controller CT which drives and stops the electric parts including the electromagnetic valves, pumps, and compressors in accordance with programs to execute various processes including wash, disinfection, rinse and drying processes.

Various types of fluid, that is, chemicals, are stored in not-shown tanks placed in the apparatus body 200. Tap water, which is for being rinse water and diluting the wash fluid and the disinfection fluid, is supplied to the endoscope water disinfector 100 via not-shown hose pipes connected from the faucet to the apparatus body 200.

Referring to Figs. 3-5, the foregoing brush cassette 9 will now be detailed.

The brush cassette 9 is an approximately cylindrical hollow container made of for example resin material. Thus, as shown In Fig. 4, this brush cassette 9 has a brush containing chamber 8 consisting of the approximately cylindrical container part. This brush containing chamber 8 has a brush delivery duct 11, a fluid supply duct 12, an air discharge duct 13, and a remaining-fluid discharge duct 14, which are integral with the chamber 8 and extend along the longitudinal direction of this chamber 8 (i.e., the axial direction which is along a later-described delivery direction X). These ducts 11-14 communicates with the brush containing chamber 8.

The brush containing chamber 8 has a longitudinal section, of which shape is approximately semi-ellipsoid as shown in Fig. 5A and provides an inner space 8i. A washing brush 7 is wound and contained in the Inner space 8i. The washing brush 7 has a long wire 7w and a brush 7b (i.e., brush element) fixed to the tip of the wire 7w wound in the inner space 8i. The wound wire 7w can be expanded and contracted so as to push forward and pull back the brush 7b in the delivery direction.

In the brush containing chamber 8, there are provided a pair of rollers 10 serving as a washing-brush drive member. Fig. 5A shows only one roller. When the brush cassette 9 is loaded in the brush-cassette loading section 150, the rollers 10 are rotatable by a not-shown rotatable roller shaft, which water-tightly stands up from the bottom of that loading section 150 into the inner space 8i through a not-shown opening formed in the bottom of the brush containing chamber 8. The not-shown roller shaft is driven by a not-shown electric motor. In reply to a command from the controller CT, the motor is driven to rotate the roller shaft in clockwise and counterclockwise directions in a selected manner.

The paired rollers 10 _pinch the wire 7w of the washing brush 7. Hence, in reply to the rotation of the roller shaft, the rollers 10 are able to deliver the washing brush 7 from the inner space 8i toward the therapeutic-instrument insertion duct (one of the endoscopic channels) of the endoscope 2 via the brush delivery duct 11, the fluid-supply nozzle 15, and the opening 5 of the endoscope 2 and, in contrast, pull back the washing brush 7 from the therapeutic-instrument insertion duct to the Inner space 81 of the chamber 8. In other words, the washing brush 7 can reciprocate between the inner space 8i of the chamber 8 and the therapeutic-instrument insertion duct.

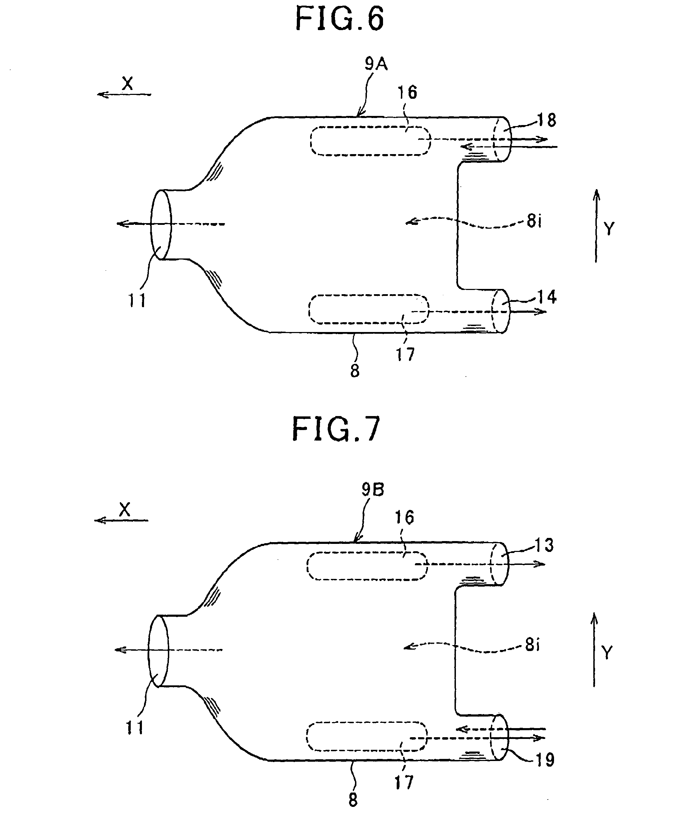

As shown In Fig. 6, the delivery direction X is assigned to the direction along which the washing brush 7 is delivered from the brush containing chamber 8, and a radial direction Y is thus assigned to a chamber radial direction perpendicular to the delivery direction X. When considering this directional relationship, it is defined that the brush deliver duct 11 is formed to protrude from the end of the brush containing chamber 8 in the delivery direction X and located at an approximately central position of the chamber 8 in the radial direction Y.

When the brush cassette 9 is loaded in the brush-cassette loading section 150, the brush delivery duct 11 is positioned to face the opening 5 of the therapeutic-instrument insertion duct of the manipulating device 2s of the endoscope 2 mounted in the manipulating-device mounting section 160 of the washing/disinfecting bath 1. Preferably, the brush delivery duct 11 is positioned to be coaxial with the opening 5.

Moreover, when loading the brush cassette 9 into the brush-cassette loading section 150, the brush delivery duct 11 is structured such that this duct 11 Is water-tightly connected to the fluid-supply nozzle 15 of the brush-cassette loading section 150 automatically or with the aid of operator's manual work. The connection between the brush delivery duct 11 and the fluid-supply nozzle 15 may be made by using a tube.

As stated, the brush deliver duct 11 is formed to communicate with the inner space 8i of the brush containing chamber 8. Thus, when the washing brush 7 and chemicals are fed from the inner space 8i, these washing brush 7 and chemicals are delivered and supplied, into the therapeutic-instrument insertion duct of the endoscope 2 by way of the brush delivery duct 11, fluid-supply nozzle 15, and opening 5. Hence the washing brush 7 can provide slide motions, in association with the chemicals, In and through the therapeutic-instrument insertion duct, so that the duct can be washed and disinfected.

In contrast, the fluid supply duct 12 is located on the opposite side (i.e., base end side) of the brush containing chamber 8 from the brush delivery duct 11 in the delivery direction X. Specifically, the fluid supply duct 12 is located on the base end side in the delivery direction X, but at an approximately central poison in the radial direction Y, and is formed to protrude toward the base end side.

When the brush cassette 9 is loaded to the brush-cassette loading section 150, the fluid supply duct 12 is connected water-tightly to a not-shown chemical supply duct in which the pumps for the wash fluid, disinfection fluid, and others are inserted, in an automatic fashion or by operator's manual work. As a modification, the connection between the fluid supply duct 12 and the not-shown chemical supply duct can be made using a tube, for example.

As stated, the fluid supply duct 12 is also formed to communicate with the inner space 8i of the brush containing chamber 8. Accordingly, driving assigned pumps arranged in the apparatus body 200 makes it possible that the wash fluid and the disinfection fluid are supplied, separately from each other, from a wash fluid tank and a disinfection fluid tank to the inner space 8i of the chamber 8 via the not-shown chemical supply duct and the fluid supply duct 12. Both tanks are provided within the apparatus body 200.

Further, the air discharge duct 13 is located on the base end side In the delivery direction X, and located at an upper position in the radial direction Y, and is formed to protrude from the brush containing chamber 8 toward the base end side. In detail, in its used state, this air discharge duct 13 is located at the highest radial position, which is higher than the fluid supply duct 12. In the state where the brush cassette 9 is loaded in the brush-cassette loading section 150, the air discharge duct 13 has the same radial position as the upper-side inner wall of the chamber 8.

When loading the brush cassette 9 to the brush-cassette loading section 150, the air discharge duct 13 is water-tightly connected to a not-shown circulation path that communicates with the washing/disinfecting bath 1 automatically or with the help of operator's manual work. Of course, the air discharge duct 13 may be connected with the not-shown circulation path using a tube. As stated, this duct 13 also communicates with the inner space 8i of the brush containing chamber 8.

The chemicals inflowing into the brush containing chamber 8 involve air therein, so that the air 16 is obliged to accumulate in an upper region in the inner space 81 of this chamber, as pictorially shown in Fig. 5A. The air discharge duct 13 is a duct for discharging the accumulated air 16 outside the chamber 8. That is, the duct 13 discharges the accumulated air 16 into the washing/disinfecting bath 1. This air 16 accumulates during not only the supply of the chemicals to the chamber 8 via the fluid supply duct 12 but also the supply of the chemicals reserved in the chamber 8 from the brush delivery duct 11 to the therapeutic-instrument insertion duct via the fluid-supply nozzle 15 and the opening 5. This supply is performed for washing and disinfecting the therapeutic-instrument Insertion duct of the endoscope 2. The reason why the air accumulates as stated above is that the volume of the inner space 8i of the brush containing chamber 8 is larger in comparison with the radius of the brush delivery duct 11 through which the chemicals are fed out.

The accumulated air is discharged via the duct 13 due to its light specific gravity. By discharging the air like this, all the inner space 8i of the brush containing chamber 8 can be filled with a chemical when the chemical is supplied to this chamber 8. It is thus possible to exclude a region in which no chemical exists. That is, the air discharge duct 13 is used to discharge the air 16, thus filling all the inner space 8i with the chemical to be supplied. As a result, the entire portion of the wire 7w can be immersed completely in the chemical.

As shown in Fig. 5A, the remaining-fluid discharge duct 14 is located on the base end side of the brush containing chamber 8 in the delivery direction X, but located at a lower position in the radial direction Y, and is formed to protrude from the chamber 8. In detail, in its use state, the duct 14 is located at the lowest position of the brush containing chamber 8, which is thus lower than the fluid supply duct 12. Accordingly, when the brush cassette 9 is loaded into the brush-cassette loading section 150, the remaining-fluid discharge duct 14 keeps the same radial level as the lower-side inner wall of the chamber 8.

When the brush cassette 9 is loaded to the brush-cassette loading section 150, the remaining-fluid discharge duct 14 is connected water-tightly to a not-shown circulation path which communicates with the washing/disinfecting bath automatically or with the help of operator's manual work. This duct 14 may be connected to the not-shown circulation path using a tube. As stated, the duct 14 also communicates with the inner space 8i of the brush containing chamber 8.

The purpose of forming the remaining-fluid discharge duct 14 is to discharge a chemical 17 which remains in an lower portion in the inner space 8i, outside the inner space 8i, specifically, to the washing/disinfecting bath 1. When washing and disinfecting the therapeutic-instrument insertion duct is finished and then supplying a chemical to the brush containing chamber 8 via the fluid supply duct 12 is finished, the chemical inevitably remains In the inner space 8i of the chamber as a remaining chemical 17, as pictorially illustrated in Fig. 5A. The reason for occurrence of the remaining chemical is that the brush cassette 9 is loaded in almost horizontally in the brush-cassette loading section 150 and the bush delivery duct 11 is located at the approximately central part of the chamber 8 in the radial direction Y. In the present embodiment, this remaining chemical is discharged via the remaining-fluid discharge duct 14, when the wash and disinfection is ended. Because the duct 14 is located at the lowest position, the remaining fluid leaks out from the duct 14.

Accordingly, when the therapeutic-Instrument insertion duct has been washed and disinfected, the disposal type of brush cassette 9 is picked up from the brush-cassette loading section 15. During this picking-up operation, the conventional drawback that the remaining chemical 17 in a lower part of the inner space 8i of the chamber 8 abruptly leaks out does not occur.

In this way, the brush containing chamber 8 of the brush cassette 9 is provided with, in addition to the brush delivery duct 11 and the fluid supply duct 12, the air discharge duct 13 and the remaining-fluid discharge duct 14. The air discharge duct 13 is able to discharge the air 16 accumulated in an upper part of the inner space 8i during the supply of the chemicals. The remaining-fluid discharge duct 14 is able to discharge, from the inner space 81, the chemical 17 remaining in a lower part of the inner space 8i, when the supply of the chemical is ended.

Thus it is possible to reliably discharge, to the outside of the inner space 8i, the air in the inner space 81 through the air discharge duct 13. When the washing brush 7 is washed and disinfected in the inner space 8i, the washing brush 7 can be immersed In the chemicals in a reliable manner. It is therefore possible that the foregoing conventional difficulty that a part of the washing brush 7, which is located in an upper part of the inner space 8i in which the air 16 is accumulated, is not touching or Incompletely touching the chemicals, is prevented from reducing the washing capability or the disinfection capability. Unlike the conventional, it is therefore unnecessary to remove the brush cassette 9 from the brush-cassette loading section 150 to again wash and disinfect the washing brush 7 accommodated in the removed brush cassette 9 in a separate manner.

In addition, even if the brush cassette 9 is horizontally loaded in the brush-cassette loading section 150, the remaining chemical 17 in the chamber 8 is discharged outside via the remaining-fluid discharge duct 14 when the supply of the chemicals ends. Thus, during picking up the brush cassette 9 from the brush-cassette loading section 150, no chemical leaks out from the brush delivery duct 11, thereby facilitating the picking-up work of the brush cassette 9.

Therefore, in the present embodiment, the brush cassette and the endoscope washer disinfector according to the present embodiment allows the chemicals to be supplied reliably to the washing brush 7 accommodated in the brush containing chamber 8, so that the washing brush 7 can be washed and disinfected in a reliable manner. Further, no remaining chemicals occur when supplying the chemicals is finished, so that the brush cassette can be removed more easily.

As a modification, the shape of the brush cassette 9 is not limited to the foregoing approximately cylinder shown In Fig. 5A. For example, as shown in Fig. 5B, the brush cassette 9 may be structured such that, at least, the inner radius expands as advancing toward the base end side in the delivery direction X. This expanding structure of the inner diameter enhances the discharge performance of the accumulated air 16 and the remaining chemical 17. Concerning this diameter expanding structure, both inner and outer diameters of the body, that is, the thickness itself of the cassette body may be increased toward the base end side.

### (Modifications)

Various modifications of the foregoing embodiment will now be described. In the following, the components identical or similar to those in the foregoing embodiment will be given the same reference numerals, with their explanations being simplified or omitted.

Fig. 6 shows a brush cassette 9A according to a modification.

In the foregoing embodiment, the brush delivery duct 11, the fluid supply duct 12, the air discharge duct 13, and the remaining-fluid discharged duct 14 are produced to be located at different positions of the brush containing chamber 8.

However, this is not always a definitive example, but as shown in Fig. 6, the fluid supply duct 12 and the air discharge duct 13 can be unified Into a single fluid-supply/air-discharge duct 18. This fluid-supply/air-discharge duct 18 is the same as the foregoing air discharge duct 13 concerning the connected position to the brush containing chamber 8.

In this configuration, there can be provided the similar advantages to those obtained in the foregoing embodiment. Besides, the number of duct connection points of the brush cassette 9A to the apparatus body 200 is reduced from four to three, differently from the foregoing embodiment. Thus the watertight performance of the brush cassette 9A to the brush-cassette loading section 150 can be enhanced.

Fig. 7 shows a brush cassette 9B according to another modification.

In the configuration shown in Fig. 7, the foregoing fluid supply duct 12 and the remaining-fluid discharge duct 14 are unified as a single fluid-supply/remaining-fluid-discharge duct 19. This fluld-supply/remaining-fluid-discharge duct 19 is the same as the foregoing remaining-fluid discharged duct 14 concerning the connected position to the brush containing chamber 8.

This modification also provides the identical operations and advantages to those gained by the modification shown in Fig. 6.

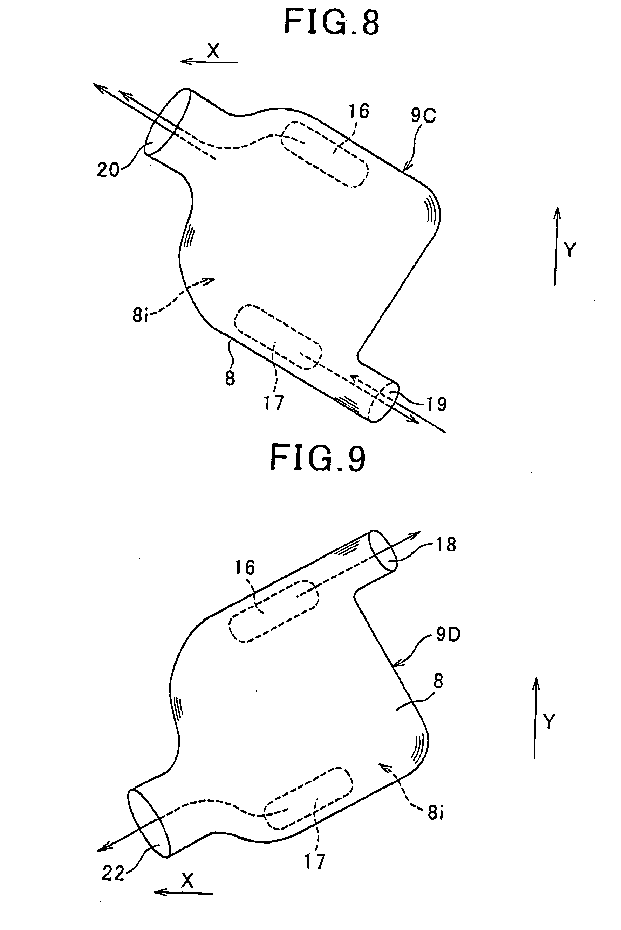

Fig. 8 shows a brush cassette 9C according to another modification.

As shown in Fig. 8, the fluid supply duct 12 and the remaining-fluid discharge duct 14 are unified as a single fluld-supply/remalning-fluid-discharge duct 19, while the brush delivery duct 11 and the air discharge duct 13 are unified a single brush-delivery/air-discharge duct 20.

In this configuration, the fluid-supply/remaining-fluid-discharge duct 19 is the same as the foregoing remaining-fluid discharge duct 14 as to its connected point to the brush containing chamber 8. The brush-delivery/air-discharge duct 20 is the same as the brush delivery duct 11 as to its connected point to the chamber 8.

In addition, the brush cassette 9C is detachably loaded to the brush-cassette loading section 150 in such a manner that the brush-delivery/air-discharge duct 20 is upward oblique to the distal end side in the delivery direction X. Thus the brush-delivery/air-discharge duct 19 is directed to be downward oblique in the delivery direction X. In the brush-cassette loading section 150, a not-shown groove is formed, which accepts the brush cassette 9C such that the distal side of the brush cassette 9C is directed in the upward oblique direction. In this upward oblique attitude, the brush-dellvery/air-discharge duct 20 is connected to the fluid-supply nozzle 15 with a tube, for example.

In this modification, the similar advantages to those gained in the foregoing embodiment are also obtained. In addition, the brush cassette 9C is loaded obliquely, so that the position of the duct 20 in the radial direction Y and the position of the accumulated air can be made agree to each other. Thus, in discharging the air, it is easier to make use of the fact that the air is smaller in specific gravity than the chemicals. The remaining chemical 17 is discharged from the duct 19 due to its weight. It is therefore possible that the duct connection points of the brush cassette 9C to the apparatus body 200 can be reduced down to two points, differently from the four points in the foregoing embodiment, enhancing the watertight performance of the brush cassette 9C to the brush-cassette loading section 150.

Fig. 9 shows a brush cassette 9D according to another embodiment.

As shown in Fig. 9, the fluid supply duct 12 and the air discharge duct 13 are unified as a fluid-supply/air-discharge duct 18, whilst the brush delivery duct 11 and the remaining-fluid discharge duct 14 are unified as a brush-delivery/remaining-fluid-discharge duct 22.

In this case, the fluid-supply/air-discharge duct 18 is the same as the foregoing air discharge duct 13 as to its connected position to the chamber 8. The brush-delivery/remaining-fluid-discharge duct 22 is located at the same connected position to the chamber as the foregoing brush delivery duct 11.

The brush cassette 9D is detachably loaded to the brush-cassette loading section 150 in the attitude which the brush-delivery/remaining-fluid-discharge duct 22 is downward oblique to the distal end side in the delivery direction X and the fluid-supply/air-discharge duct 18 Is upward oblique to the rear end side in the delivery direction X. The brush-cassette loading section 150 has a not-shown groove to accept the brush cassette 9D in such a downward oblique attitude. With this downward oblique attitude kept, the brush-delivery/remaining-fluid-discharge duct 22 is connected to the fluid-supply nozzle 15 with a tube, for example.

Thus the brush cassette 9D is able to provide the duct configuration that makes use of the difference of specific gravity between the air and the chemicals, providing the similar operations and advantages to those gained in the foregoing embodiment and modification shown in Fig. 8.

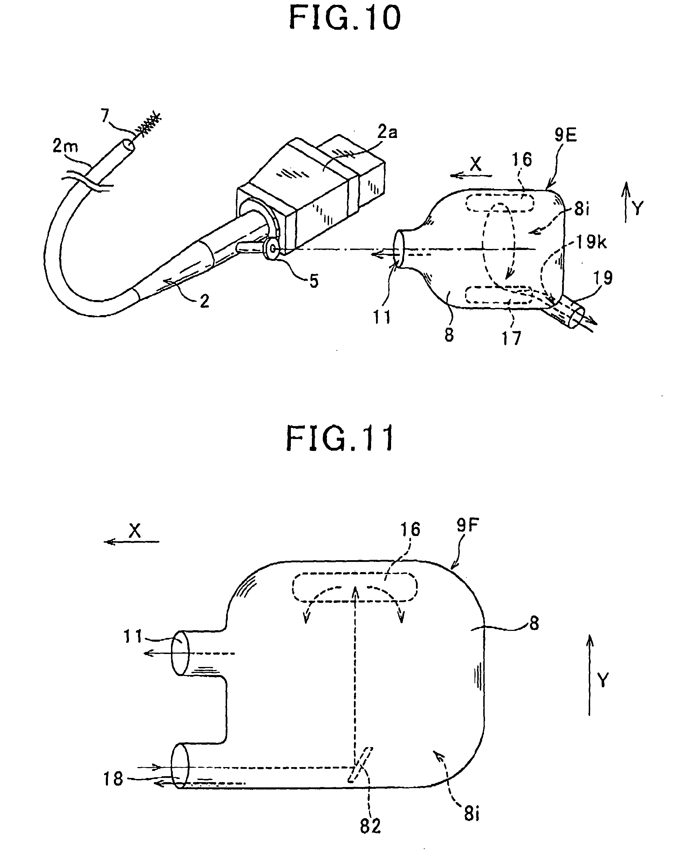

Fig. 10 shows a brush cassette 9E according to another modification. In Fig. 10, the brush cassette 9E is shown together with an endoscope.

As shown in Fig. 10, the brush cassette 9E is loaded to the brush-cassette loading section 150 such that the brush delivery duct 11 is located coaxially to the opening 5 of the therapeutic-instrument insertion duct formed through the manipulated device 2s of the endoscope 2. However, in this case, as stated before, the air 16 accumulates in an upper region in the Inner space 8i of the brush containing chamber 8, while the chemical 17 remains in a lower portion in the inner space 8i of this chamber 8.

With consideration of these circumstance, as shown In Fig. 10, the fluid supply duct 12 and the remaining-fluid discharge duct 14 are unified into the single fluld-supply/remaining-fluid-discharge duct 19. In this case, this duct 19 is secured to a lower side of the chamber 8. This duct 19 is oblique to both directions X and Y and this duct 19 has an opening 19k opened to the inner space 8i and directed in a circular tangential direction provided by the cylindrical wall face of the inner space 8l.

In this configuration, after finishing the supply of the chemicals, the remaining chemicals 17 in the lower portion of the chamber 8 are discharged through the fluid-supply/remaining-fluid-discharge duct 19. Further, the opening 19k is directed in the tangential direction of the Inner cylindrical wall, so that the chemicals are supplied along the tangential direction through this duct 19. With no air discharge duct 13 formed to the chamber 8, it is therefore possible to allow the chemicals to circulate through the air accumulated region and to run along the inner wall In a spiral form in the inner space 8i. Thus the accumulated air is flushed away or removed, resulting in overcoming the drawback due to the air accumulation and providing a reliable wash and disinfection to the washing brush 7.

Furthermore, the number of duct connection points of the brush cassette 9E to the apparatus body 200 is reduced down to two. This provides a higher watertight performance between the brush cassette 9E and the brush-cassette loading section 150.

Fig. 11 shows a brush cassette 9F according to another modification.

As shown in Fig. 11, the fluid supply duct 12 and the remaining-fluid discharge duct 14 are unified as the single fluid-supply/remaining-fluid-discharge duct 19. This duct 19 is connected to the lowest position of the brush cassette 9F in the radial direction Y. Specifically, the fluid-supply/remaining-fluid-discharge duct 19, in addition to the brush delivery duct 11. Specifically, both the ducts 19 and 11 are produced to protrude from the distal end of the brush containing chamber 8 in the same way In the delivery direction X.

Additionally, in the cylindrical inner space 8i of the chamber 8, there is provided a deflection plate 82 serving as a deflection member to deflect the chemicals supplied through the duct 19 toward an upper air accumulated region in the inner space 8i. This deflection plate 82 is a plate-like member secured on the bottom of the inner space 8i and the area, located position, and located angle of this plate 82 are set to make it easier that the deflected chemicals are directed to the position where the air is likely to accumulate. The deflection plate 82 may have a curved face to receive the chemicals.

After finishing the supply of the chemicals, the remaining chemical 17 in the lower region of the chamber 8 is discharged through the fluid-supply/remaining-fluid-discharge duct 19. Meanwhile the chemicals received through the duct 19 is hit onto the deflection plate 81 in the inner space 8i. Each of the supplied chemicals has a predetermined pressure, so that the pressure of each chemical enables the supplied chemical to be deflected toward the region where the air is likely to accumulate. Hence the deflected chemical is forced to reliably pass through this region. This deflection operation will cause the chemical to circulate through every region in the inner space 8i so as to not produce the air accumulation. Without connecting the air discharge duct 13, each chemical can be sent to every region in the inner space 8i without fail, so that every portion of the washing brush 7 can be washed and disinfected.

After the brush cassette 9F is loaded to the brush-cassette loading section 150, the operator is able to connect, to the apparatus body 200, both the ducts 19 and 11 in the same direction. This makes it possible to easily load the brush cassette 9F, improving efficiency of the wash and disinfection work.

Furthermore, the duct connection points of the brush cassette 9F to the apparatus body are kept to be two. Hence it is easier to secure the watertight performance between the brush cassette 9 and the brush-cassette loading section 150.

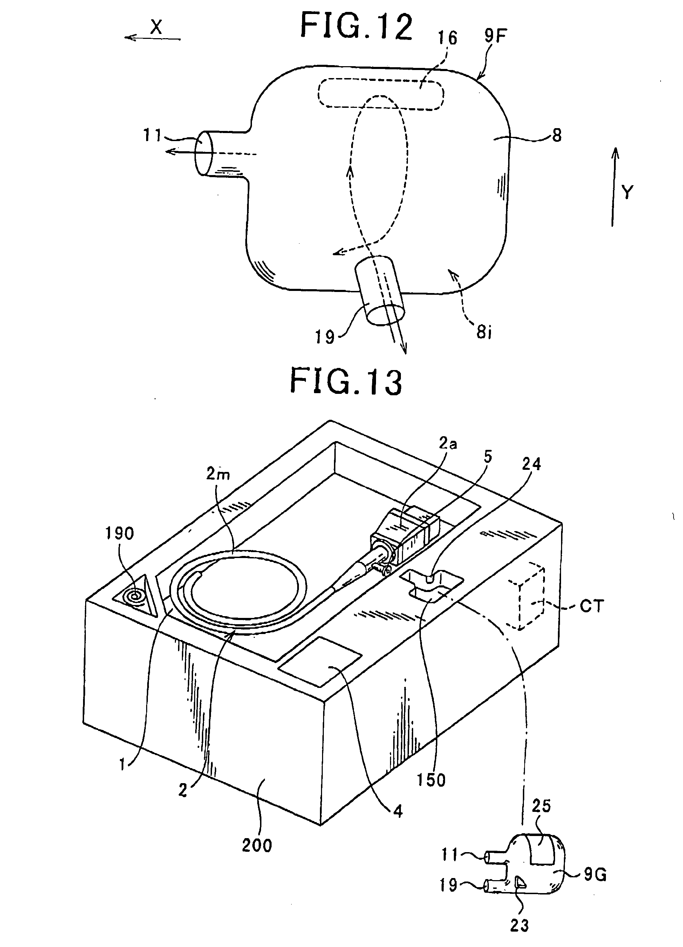

Fig. 12 shows a brush cassette 9G according to another modification. This brush cassette 9G is developed form the brush cassette 9F shown In Fig. 11. The brush cassette 9G shown in Fig. 12 is provided with the brush delivery duct 11 and the fluid-supply/remaining-fluid-discharge duct 19, which communicate with the brush containing chamber 8. Of there ducts, the brush delivery duct 11 is made to protrude form the distal end face of the chamber In the delivery direction X. In contrast, the fluid-supply/remaining-fluid-discharge duct 19 is made to protrude laterally from a side of chamber 8 along a lateral direction perpendicular to the delivery direction X. Additionally to this, the protruding position of the duct 19 from the chamber 8 is set to a lower end of the side thereof. The opening of this duct 19 to the inner space 8i is directed along a tangential direction given by the cylindrical inner wall face of the chamber 8 (when viewed along the delivery direction X). Thus the flow of each chemical is forced to circulate along the cylindrical inner wall as illustrated by an arrow.

The opening of the duct 19 is positionally located, in the delivery direction X, to correspond to a region where the air is apt to accumulate. This eliminates use of the foregoing deflection plate 82. Without using such a plate, the chemicals can be circulated through every inner region in the chamber 8. Hence, like the modification shown in Fig. 11, the drawback resulting from the air accumulation can be avoided without fail.

By the way, in the brush cassette 9F, the opening of the duct to the inner space 8i is not limited to that shown in Fig. 12, but may be set to any position as long as the flow of the chemicals is circulated along the inner wall face. For example, with the brush cassette 9F loaded, the duct 19 may be protruded downward or obliquely to the delivery direction Y.

In the foregoing embodiment and its various modifications, the endoscopic duct (channel) has been exemplified as being the therapeutic-instrument insertion duct. However, this is just a possible example. The endoscopic duct to be washed and disinfected is not limited to the therapeutic-instrument insertion duct, but may be an air-supply/water-supply duct, a forward water-supply duct, or a suction duct.

By the way, the diameters of plural endoscopic ducts of the endoscope 2 are different from one another, duct by duct. When loading the brush cassette to the brush-cassette loading section 150 for washing and disinfecting the endoscope, it is required for an operator to select a washing brush 7 of which brush 7b has a diameter proper for the diameter of each duct. In addition, it is required to load the brush cassette with the selected washing brush 7 to the brush-cassette loading section 150. Inserting a washing brush equipped with an improper-diameter brush 7b into an endoscopic duct makes it difficult to insert the bush 7b into the duct or results in a poor-quality washing performance.

Accordingly, the operator has to select the washing brush 7 having a brush 7b of which diameter is appropriate for a duct diameter. For that, the operator has to pay much attention to model types or others of the endoscope and brush cassette every time the wash and distinction is performed. This results in operator's troublesome work. Hence there is a demand that the brush cassette with a proper washing brush 7b is set easily and reliably.

Fig. 13 shows a configuration provided in consideration of the above demand.

As shown in Fig. 13, on the outer surface of the brush cassette 9G, there is provided a protrusion 23 having the shape changed depending on the diameter of the brush 7b of the washing brush 7. A limit sensor 24 is placed at a potion of the brush-cassette loading section 150, where the position Is allowed to touch the protrusion 23 of the brush cassette 9G, when loading the brush cassette 9G to this loading section 150 of the apparatus body 200. The limit sensor 24 is for interpreting the diameter of the brush 7b by sensing the shape of the protrusion 23.

In addition, the endoscope 2 is equipped with a not-shown RFID (radio frequency identification) device in which the individual information of this endoscope 2, in other words, information indicative of the radii of the plural endoscopic ducts of the endoscope 2, is provided. The apparatus body 200 is a read-out device 190 with an RFID antenna to read out the individual information from the RFID device provided in the endoscope 2 mounted in the bath 1.

Moreover, the apparatus body 200 is provided with the controller CT including a computer system activated on software processing. The controller CT uses results detected with the help of the read-out device 190 and the limit sensor 24, to determine whether or not the diameter of the brush 7b is appropriate for the wash of an endoscopic duct to be washed. If not proper for that, the controller CT notifies the operator of an error using the operation panel 4 on the apparatus body 200.

In this configuration, the shape or presence of the protrusion 23 makes it possible to quickly determine whether or not the diameter of the brush 7b of the brush cassette 9G which has been set is proper. Thus, the operator is able to recognize the determined results appearing on the operation panel 4 in a short time.

As shown in Fig. 13, on the outer surface of the brush cassette 9G, a label may be provided, whose color depends on the diameter of the brush 7b. In this case, the limit sensor 24 is replaced by a color sensor that senses the label 25, in which the color senor gives the controller CT sensed information.

Using results detected by the read-out device 190 and the color sensor, the controller CT determines whether or not the diameter of the brush 7b is proper for an endoscopic duct to be washed and disinfected. When the controller CT determines failure in selecting the brush 7b, that is, the washing brush 7, this fact is notified to the operator using the operation panel 4 and another label color showing a proper brush 7b is represented on the operation panel 4.

As stated, utilizing the notifications or the label colors provided on the operation panel 4, the operator, the operator is able to set the brush cassette 9G with the proper-diameter brush 7b in an easier and reliable manner.

In this modified configuration, a symbol marking may be applied to the outer surface of the brush cassette 9G, where the symbol marking shows the foregoing individual information, not limited to the label 25. In this case, the sensor 24 is replaced by a sensor that senses the shape of the symbol marking.

Further, an RFID device may be arranged in the brush cassette 9G. In this alternative example, the sensor 24 may be a sensor, such as a magnetic sensor, for reading out the RFID.

Although the descriptions above contain many specificities, these should not be construed as limiting the scope of the invention but as merely providing illustrations of some of the presently preferred embodiments of the present invention. Thus the scope of the present invention should be determined by the appended claims.

## Claims

1. A brush cassette detachably loaded to an apparatus that provides a bath for washing and disinfecting a duct of an endoscope accommodated in the bath, comprising:
a washing brush;
a chamber accommodating the washing brush;
a brush delivery duct that communicates with the chamber and allows the washing brush to pass therethrough toward the duct of the endoscope;
a fluid supply duct that communicates with the chamber and allows a chemical for washing and disinfecting the duct to pass therethrough toward the chamber;
a brush drive member used for moving back and forth the washing brush between the chamber and the duct of the endoscope via the brush delivery duct;
a preventing means that prevents air from accumulating in the chamber; and
a remaining-fluid discharge duct that communicates with the chamber and allows the chemical remaining in the chamber to be discharged outside therethrough.

2. The brush cassette of claim 1, wherein the preventing means is an air discharge duct that communicates with the brush containing member and allows air accumulated in the brush containing member to be discharged outside the chamber.

3. The brush cassette of claim 2, wherein the duct of the endoscope has an opening and the brush delivery duct is located at a position of the chamber when the brush cassette is loaded to the apparatus, the position facing the opening of the duct of the endoscope.

4. The brush cassette of claim 3, wherein the brush delivery duct is located coaxially with the opening of the duct of the endoscope.

5. The brush cassette of claim 2, wherein the fluid supply duct and the air discharged duct are composed of a unitary duct and the unitary duct is located to an upper-end-side position of the chamber when the brush cassette is loaded to the apparatus.

6. The brush cassette of claim 2, wherein the fluid supply duct and the remaining-fluid discharge duct are composed of a unitary duct and the unitary duct is located to a lower-end-side position of the chamber when the brush cassette is loaded to the apparatus.

7. The brush cassette of claim 2, wherein the brush delivery duct and the air discharge duct are composed of a unitary duct and the brush cassette is loaded to the apparatus so that the unitary duct is directed obliquely and upward to a delivery direction of the washing brush to the duct of the endoscope.

8. The brush cassette of claim 2, wherein the brush delivery duct and the remaining-fluid discharge duct are composed of a unitary duct and the brush cassette is loaded to the apparatus so that the unitary duct is directed obliquely and downward to a delivery direction of the washing brush to the duct of the endoscope.

9. The brush cassette of claim 1, wherein the fluid supply duct and the remaining-fluid discharge duct are composed of a unitary duct and the unitary duct is located to a lower-end-side position of the chamber when the brush cassette is loaded to the apparatus.

10. The brush cassette of claim 9, wherein the chamber has a cylindrical inner space formed by a cylindrical inner wall and
the preventing means is structured such that the unitary duct has an opening opened to the inner space and the opening of the unitary duct is directed along a tangential direction of the cylindrical inner wall of the chamber.

11. The brush cassette of claim 6, wherein the unitary duct is located to be directed in the same direction as and in parallel with the brush delivery duct and
the preventing means is a deflection member arranged in the chamber and formed to deflection the chemical supplying from the unitary duct toward an upper region of the inner space where the air is to accumulate.

12. The brush cassette of claim 11, wherein the chamber has a longitudinal direction and both ends in the longitudinal direction, and
the brush delivery duct and the unitary duct are formed at one of both ends of the chamber.

13. The brush cassette of claim 12, wherein the brush delivery duct is located at a central part of the one end in a direction perpendicular to the delivery direction, and
the unitary duct is located at the one end so as to face the deflection member.

14. The brush cassette of claim 13, wherein the chamber is formed in a substantially cylindrical shape.

15. An apparatus comprising
a bath in which an endoscope having a duct is accommodated and
a detachable brush cassette having a washing brush used for washing and disinfecting the duct of the endoscope accommodated in the bath,
the brush cassette comprising
a chamber accommodating the washing brush;
a brush delivery duct that communicates with the chamber and allows the washing brush to pass therethrough toward the duct of the endoscope;
a fluid supply duct that communicates with the chamber and allows a chemical for washing and disinfecting the duct to pass therethrough toward the chamber;
a brush drive member that moves back and forth the washing brush between the chamber and the duct of the endoscope via the brush delivery duct;
a preventing member that prevents air from accumulating in the chamber; and
a remaining-fluid discharge duct that communicates with the chamber and allows the chemical remaining in the chamber to be discharged outside therethrough.

16. The apparatus of claim 15, wherein the preventing member is an air discharge duct that communicates with the brush containing member and allows air accumulated in the brush containing member to be discharged outside the chamber.

17. The apparatus of claim 16, wherein the fluid supply duct and the remaining-fluid discharge duct are composed of a unitary duct and the unitary duct is located to a lower-end-side position of the chamber when the brush cassette is loaded to the apparatus.

18. The apparatus of claim 17, wherein the unitary duct is located to be directed in the same direction as and in parallel with the brush delivery duct and
the preventing means is a deflection member arranged in the chamber and formed to deflection the chemical supplying from the unitary duct toward an upper region of the Inner space where the air is to accumulate.
